(19) **Europäisches Patentamt / European Patent Office / Office européen des brevets**

(11) **EP 4 379 740 B1**

(12) **EUROPEAN PATENT SPECIFICATION**

(45) Date of publication and mention
of the grant of the patent:
**14.01.2026   Bulletin 2026/03**

(21) Application number: **22856137.9**

(22) Date of filing: **08.08.2022**

(51) International Patent Classification (IPC):
*G16H 50/50* (2018.01)          *G16H 50/70* (2018.01)
*G16H 50/20* (2018.01)          *G16H 10/60* (2018.01)
*G16H 30/40* (2018.01)          *A61B 34/10* (2016.01)
*A61B 5/103* (2006.01)          *A61B 5/00* (2006.01)
*G06T 17/00* (2006.01)          *G06T 7/70* (2017.01)
*G09B 23/28* (2006.01)          *G06T 7/00* (2017.01)
*G16H 20/40* (2018.01)

(52) Cooperative Patent Classification (CPC):
**G16H 30/40; A61B 34/10; G06T 7/0012;
G09B 23/28; G09B 23/285; G16H 10/60;
G16H 20/40; G16H 50/20; G16H 50/50;
G16H 50/70;** A61B 2034/105; G06T 2207/10081;
G06T 2207/10088; G06T 2207/10104;
G06T 2207/20081;                              (Cont.)

(86) International application number:
**PCT/KR2022/011757**

(87) International publication number:
**WO 2023/018138 (16.02.2023 Gazette 2023/07)**

(54) **DEVICE AND METHOD FOR GENERATING VIRTUAL PNEUMOPERITONEUM MODEL OF PATIENT**

VORRICHTUNG UND VERFAHREN ZUR ERZEUGUNG EINES VIRTUELLEN PNEUMOPERITONEUMMODELLS EINES PATIENTEN

DISPOSITIF ET PROCÉDÉ DE GÉNÉRATION D'UN MODÈLE DE PNEUMOPÉRITOINE VIRTUEL D'UN PATIENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.08.2021   KR 20210105582**

(43) Date of publication of application:
**05.06.2024   Bulletin 2024/23**

(73) Proprietor: **HUTOM CO., LTD.
Seoul 04151 (KR)**

(72) Inventors:
• **HONG, Helen**
  **Seoul 06602 (KR)**
• **LEE, Min Jin**
  **Seoul 03336 (KR)**
• **HYUNG, Woo Jin**
  **Seoul 06574 (KR)**
• **KIM, Yoo Min**
  **Seoul 03032 (KR)**
• **SUNG, Nak Jun**
  **Seoul 04137 (KR)**

(74) Representative: **dompatent
Partnerschaft von
Patentanwälten und Rechtsanwälten mbB
Deichmannhaus am Dom
Bahnhofsvorplatz 1
50667 Köln (DE)**

(56) References cited:
KR-A- 20120 111 871      KR-A- 20160 092 425
KR-A- 20190 088 419      KR-A- 20200 056 855
KR-A- 20200 056 855      KR-B1- 102 013 814
KR-B1- 102 013 814

(52) Cooperative Patent Classification (CPC): (Cont.)
G06T 2207/20084

**Description**

[TECHNICAL FIELD]

Embodiments of the present disclosure described herein relate to a device and a method for generating a virtual pneumoperitoneum model of a patient.

[BACKGROUND ART]

Recently, there has been a need for a device and software for allowing medical teams to perform training in a situation similar to reality. In general, a simulation device for medical teams is manufactured to be similar to the situation of a patient to perform training. Such simulation devices do not provide several situations which occurs in the patient and has falling reality when performing simulation. Furthermore, for surgical operations, the medical team is unable to perform simulation under the same conditions as actual surgery.

Furthermore, there is a need to develop technologies capable of providing information for assisting a doctor to perform surgery, in a surgery process. To provide the information for assisting the doctor to perform surgery, it should be able to recognize surgical actions. Previously, to plan a scenario to optimize the surgical process, medical teams referred to previously captured medical imaging or received advice from a highly experienced doctor. It was difficult to determine an unnecessary process using only medical imaging and it was difficult to receive advice suitable for a specific patient from an experienced doctor. Thus, medical imaging or advice from an experienced doctor was often difficult to use for auxiliary purposes in optimizing the surgical process for the patient undergoing surgery.

When performing surgery simulation using virtual reality, only when the medical team should perform training under the same conditions as during actual surgery, the surgery simulation may serve as a rehearsal.

Particularly, when performing minimally invasive surgery (e.g., robotic surgery or laparoscopic surgery), as a difference between an image that the medical team checked during the simulation process and an image shown during actual surgery is generated when an image capture direction of a camera during actual surgery and an image capture direction of the camera during surgery simulation are different from each other, the effect of performing training to be the same as the actual surgery may not be obtained. In other words, because the intra-abdominal structure is able to vary with the position of the camera for checking the intra-abdominal structure, there is a need to implement the camera to enter the body to be the same as during actual surgery during surgery simulation.

Thus, for the camera to provide the same imaging as during actual surgery during surgery simulation, there is a need to implement a virtual pneumoperitoneum model to which a pneumoperitoneum condition (a condition in which gas is injected into the body of the patient to inflate the abdomen of the patient to facilitate surgery) to be the same as during actual surgery.

A similar method for generating a pneumoperitoneum model can be found in KR20200056855A. The method comprises the steps of: obtaining medical image data of a patient by a computer; obtaining scan data, which scans a body of the patient in a pneumoperitoneum state; and generating a pneumoperitoneum model by matching the medical image data and the scan data.

Another similar a method for manufacturing a virtual body model using a surgical video can be found in KR102013814B1. The method comprises the following steps of: extracting a feature point by detecting an organ from the surgical video including a plurality of image frames; matching and connecting the feature point between each of the plurality of video frames; grouping regions with similar internal motions in the organ based on the connected feature point and then dividing the organ into at least one cluster region; and matching the at least one cluster region on the virtual body model.

[DETAILED DESCRIPTION OF THE INVENTION]

[TECHINICAL PROBLEM]

Embodiments of the present disclosure provide a device and a method for generating a virtual pneumoperitoneum model of a patient to generate the virtual pneumoperitoneum model of the patient based on condition data, such as an age of the patient, a gender of the patient, a height of the patient, a weight of the patient, a body mass index of the patient, and whether the patient has given birth, body data, such as a ratio between a height and a width for a body of the patient, a skin circumference, a distance between the front of the body and the rear of the body, a fat area, and a muscle area, and landmark data displayed on abdominal 3D imaging data of the patient before pneumoperitoneum.

Embodiments of the present disclosure provide a device and a method for generating a virtual pneumoperitoneum model of a patient to provide a surgery simulation environment based on the virtual pneumoperitoneum model in which an actual pneumoperitoneum condition of the patient is predicted, such that the surgery simulation serves as an excellent rehearsal for actual surgery.

The technical problems to be solved by the present disclosure are not limited to the aforementioned problems, and any other technical problems not mentioned herein will be clearly understood from the following description by those skilled in the art to which the present disclosure pertains.

[TECHNICAL SOLUTION]

According to an embodiment, a method for generating a virtual pneumoperitoneum model of a patient may include obtaining condition data of the patient, the condition data including data about at least one of an age of the patient, a gender of the patient, a height of the patient, a weight of the patient, a body mass index of the patient, or whether the patient has given birth, obtaining a plurality of pieces of landmark data of the patient, the plurality of pieces of landmark data being displayed on an abdominal 3D imaging data of the patient, obtaining body data extracted from a plurality of pieces of cross-sectional imaging data of the patient, the plurality of pieces of cross-sectional imaging data are a cross section of positions at which the plurality of pieces of landmark data are displayed and the body data including at least one of a ratio between a height and a width for a body of the patient in the plurality of pieces of cross-sectional imaging data, a skin circumference, a distance between the front of the body and the rear of the body, a fat area, or a muscle area, and generating the virtual pneumoperitoneum model for predicting an actual pneumoperitoneum condition of the patient based on the condition data, the plurality of pieces of landmark data, and the body data.

At this time, the obtaining of the plurality of pieces of landmark data may include generating one piece of reference landmark data at a predetermined position with respect to a navel of the patient based on the abdominal 3D imaging data of the patient and additionally generating the plurality of pieces of landmark data on the basis of the reference landmark data.

Furthermore, the generating of the virtual pneumoperitoneum model may include selecting specific pneumoperitoneum shape data with a highest matching rate with the condition data, the landmark data, and the body data among a plurality of pieces of previously stored pneumoperitoneum shape data based on a first algorithm. In detail, the generating of the virtual pneumoperitoneum model may include selecting a specific pneumoperitoneum shape class with a highest similarity with the condition data, the landmark data, and the body data among a plurality of previously stored pneumoperitoneum shape classes, selecting the specific pneumoperitoneum shape data in the selected specific pneumoperitoneum shape class, based on the first algorithm. The plurality of previously stored pneumoperitoneum shape classes may be generated by clustering the condition data, the body data, and the pneumoperitoneum shape data for every a plurality of existing patients.

Furthermore, the generating of the virtual pneumoperitoneum model may include generating the virtual pneumoperitoneum model based on the condition data and the body data by means of a machine learning model. In detail, the generating of the virtual pneumoperitoneum model may include calculating position information after pneumoperitoneum in the landmark data for the patient by means of the machine learning model, generating a body surface of the patient based on the position information after pneumoperitoneum and outputting the virtual pneumoperitoneum model. The machine learning model may be trained based on a training dataset constructed by constructing a training dataset based on the condition data, the body data, the landmark data, and landmark data after pneumoperitoneum for every a plurality of existing patients. The landmark data after pneumoperitoneum may be obtained based on an actual pneumoperitoneum result when performing surgery on an existing patient.

Furthermore, the obtaining of the body data may include obtaining the fat area based on the plurality of pieces of cross-sectional imaging data by means of a fat extraction model and obtaining the muscle area based on the plurality of pieces of cross-sectional imaging data by means of a muscle extraction model. The fat extraction model may be a machine learning model trained by specifying only a fat area as a region of interest on abdominal medical imaging data. The muscle extraction model may be a machine learning model trained by specifying only a muscle area as a region of interest on the abdominal medical imaging data.

According to an embodiment, a device for generating a virtual pneumoperitoneum model of a patient may include a memory storing a plurality of processes for generating the virtual pneumoperitoneum model of the patient and a processor that generates the virtual pneumoperitoneum model of the patient based on the plurality of processes. The processor may obtain condition data of the patient, the condition data including data about at least one of an age of the patient, a gender of the patient, a height of the patient, a weight of the patient, a body mass index of the patient, or whether the patient has given birth, may obtain a plurality of pieces of landmark data of the patient, the plurality of pieces of landmark data being displayed on an abdominal 3D imaging data of the patient, may obtain body data extracted from a plurality of pieces of cross-sectional imaging data of the patient, the plurality of pieces of cross-sectional imaging data are a cross section of positions at which the plurality of pieces of landmark data are displayed and the body data including at least one of a ratio between a height and a width for a body of the patient in the plurality of pieces of cross-sectional imaging data, a skin circumference, a distance between the front of the body and the rear of the body, a fat area, or a muscle area, and may generate the virtual pneumoperitoneum model for predicting an actual pneumoperitoneum condition of the patient based on the condition data, the plurality of pieces of landmark data, and the body data.

In addition, another method for implementing the present disclosure, another apparatus therefor, another system therefor, and a computer-readable storage medium storing a computer program for executing the method may be further provided.

[ADVANTAGEOUS EFFECTS OF THE INVENTION]

According to the present disclosure, the device for generating the virtual pneumoperitoneum model of the patient may generate the virtual pneumoperitoneum model of the patient based on condition data, such as an age of the patient, a gender of the patient, a height of the patient, a weight of the patient, a body mass index of the patient, and whether the patient has given birth, body data, such as a ratio between a height and a width for a body of the patient, a skin circumference, a distance between the front of the body and the rear of the body, a fat area, and a muscle area, and landmark data displayed on abdominal 3D imaging data of the patient before pneumoperitoneum, thus predicting an actual pneumoperitoneum condition of the patient at high accuracy.

The effects of the present disclosure are not limited to the above-described effects and other effects which are not described herein will become apparent to those skilled in the art from the following description.

[DESCRIPTION OF THE DRAWINGS]

FIG. 1 is a drawing for describing a device for generating a virtual pneumoperitoneum model of a patient according to the present disclosure;

FIG. 2 is a drawing for describing a plurality of pieces of landmark data according to the present disclosure;

FIG. 3 is a drawing illustrating a ratio between a height and a width for a body of a patient in cross-sectional imaging data according to the present disclosure;

FIG. 4 is a drawing illustrating a skin circumference for a body of a patient in cross-sectional imaging data according to the present disclosure;

FIG. 5 is a drawing illustrating a distance between the front and the rear of a body of a patient in cross-sectional imaging data according to the present disclosure;

FIG. 6 is a drawing illustrating a fat area in cross-sectional imaging data according to the present disclosure;

FIG. 7 is a drawing illustrating a muscle area in cross-sectional imaging data according to the present disclosure;

FIG. 8 is a drawing for describing generating previously stored pneumoperitoneum shape classes according to the present disclosure;

FIG. 9 is a drawing illustrating pneumoperitoneum shape data included in a first class among pneumoperitoneum shape classes according to the present disclosure;

FIG. 10 is a drawing illustrating pneumoperitoneum shape data included in a second class among pneumoperitoneum shape classes according to the present disclosure;

FIG. 11 is a drawing illustrating pneumoperitoneum shape data included in a third class among pneumoperitoneum shape classes according to the present disclosure;

FIG. 12 is a drawing for describing generating a virtual pneumoperitoneum model based on previously stored pneumoperitoneum shape classes according to the present disclosure;

FIG. 13 is a drawing for describing generating a virtual pneumoperitoneum model according to the present disclosure; and

FIG. 14 is a flowchart illustrating a process of generating a virtual pneumoperitoneum model of a patient according to the present disclosure.

[BEST MODE]

Advantages, features, and methods of accomplishing the same in the present disclosure will become apparent with reference to embodiments described in detail below together with the accompanying drawings. However, the present disclosure may be embodied in various different forms, and should not be construed as being limited only to the illustrated embodiments. Rather, these embodiments are provided as examples so that the present disclosure will be thorough and complete, and will fully convey the scope of the present disclosure to those skilled in the art. The present disclosure may be defined by the scope of the claims.

The terms used herein are provided to describe embodiments, not intended to limit the present disclosure. In the specification, the singular forms include plural forms unless particularly mentioned. The expressions "comprise" and/or "comprising" used herein indicate existence of one or more other elements other than stated elements but do not exclude presence of additional elements. Like reference numerals designate like elements throughout the specification, and the term "and/or" may include each of stated elements and one or more combinations of the stated elements. The terms such as "first" and "second" are used to describe various elements, but it is obvious that such elements are not restricted to the above terms. The above terms are used only to distinguish one element from the other. Thus, it is obvious that a first element described hereinafter may be a second element within the technical scope of the present disclosure.

Unless otherwise defined, all terms (including technical and scientific terms) used herein have the same meaning as commonly understood by those skilled in the art to which the present disclosure pertains. Also, terms which are defined in a dictionary and commonly used should be interpreted as not in an idealized or overly formal detect unless expressly so defined.

Hereinafter, an embodiment of the present disclosure will be described in detail with reference to the accompanying drawings.

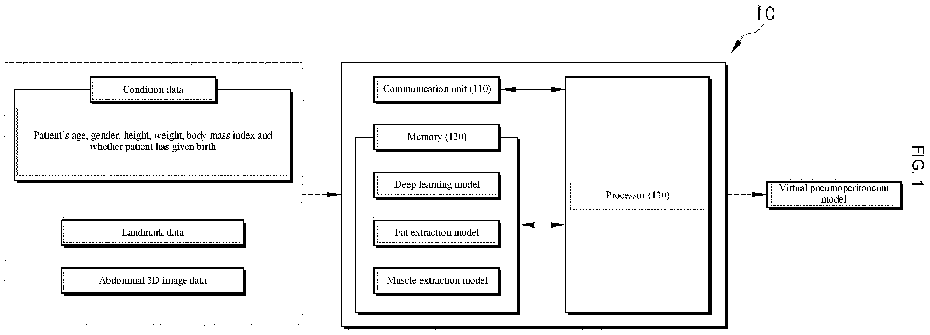

FIG. 1 is a drawing for describing a device 10 for generating a virtual pneumoperitoneum model of a patient according to the present disclosure.

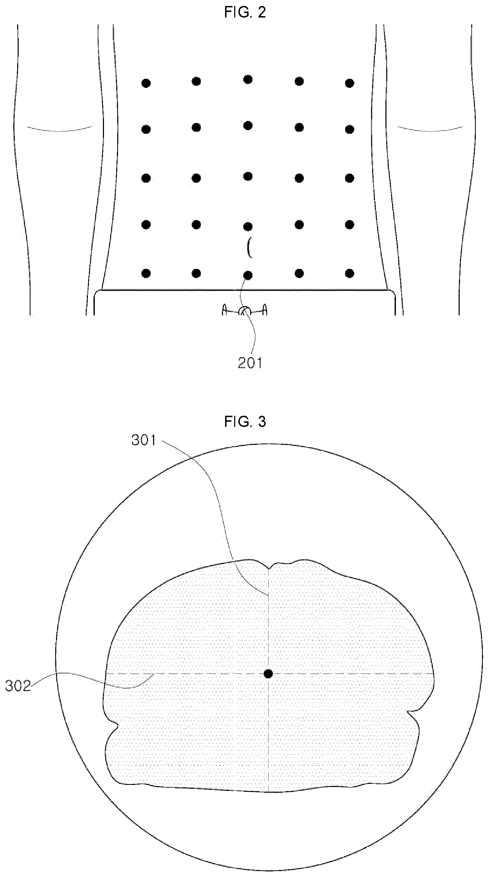

FIG. 2 is a drawing for describing a plurality of pieces of landmark data according to the present disclosure.

FIG. 3 is a drawing illustrating a ratio between a height and a width for a body of a patient in cross-sectional imaging data according to the present disclosure.

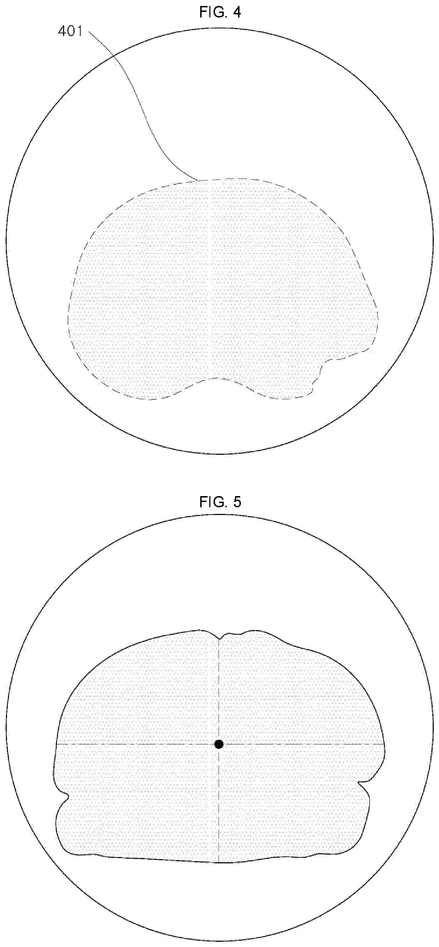

FIG. 4 is a drawing illustrating a skin circumference for a body of a patient in cross-sectional imaging data according to the present disclosure.

FIG. 5 is a drawing illustrating a distance between the front and the rear of a body of a patient in cross-sectional imaging data according to the present disclosure.

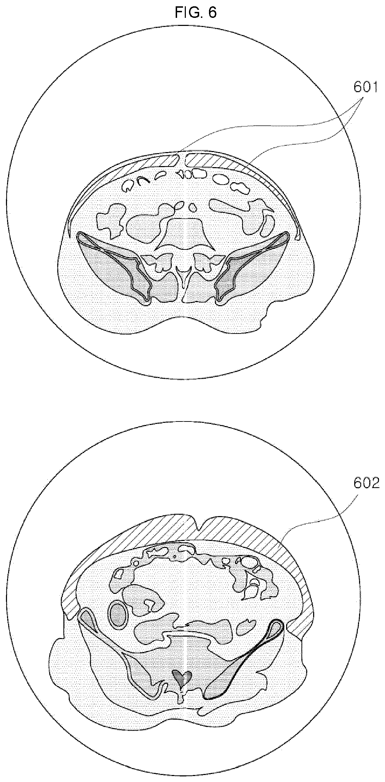

FIG. 6 is a drawing illustrating a fat area in cross-sectional imaging data according to the present disclosure.

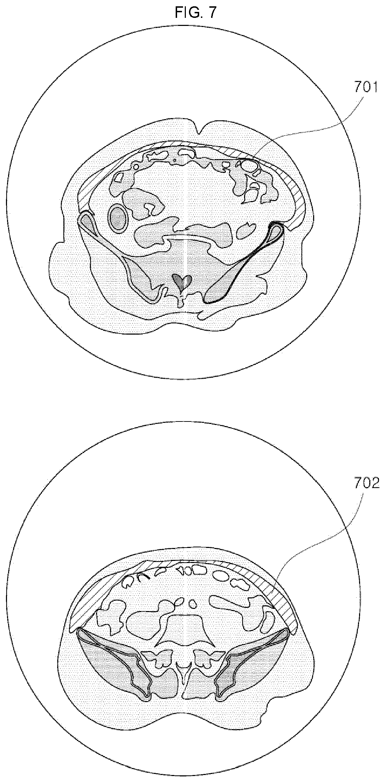

FIG. 7 is a drawing illustrating a muscle area in cross-sectional imaging data according to the present disclosure.

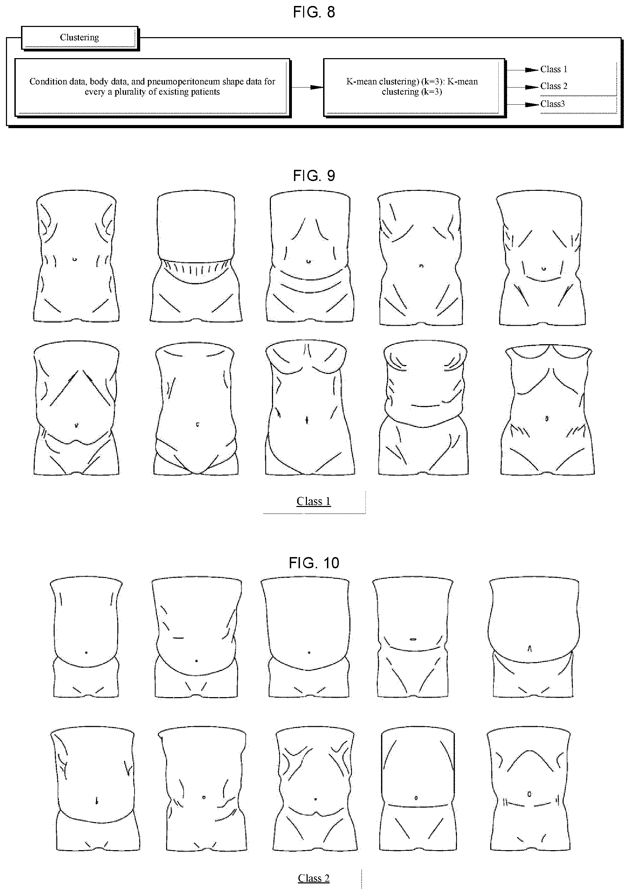

FIG. 8 is a drawing for describing generating previously stored pneumoperitoneum shape classes according to the present disclosure.

FIG. 9 is a drawing illustrating pneumoperitoneum shape data included in a first class among pneumoperitoneum shape classes according to the present disclosure.

FIG. 10 is a drawing illustrating pneumoperitoneum shape data included in a second class among pneumoperitoneum shape classes according to the present disclosure.

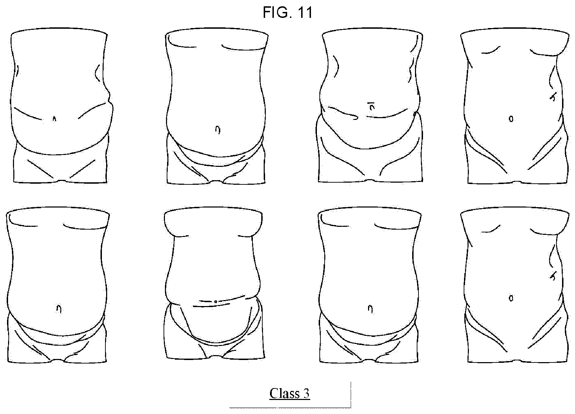

FIG. 11 is a drawing illustrating pneumoperitoneum shape data included in a third class among pneumoperitoneum shape classes according to the present disclosure.

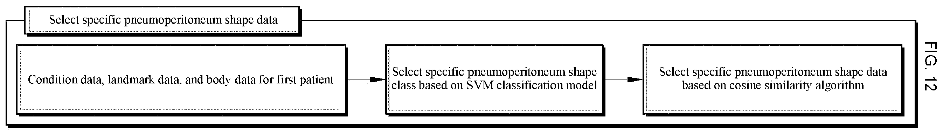

FIG. 12 is a drawing for describing generating a virtual pneumoperitoneum model based on previously stored pneumoperitoneum shape classes according to the present disclosure.

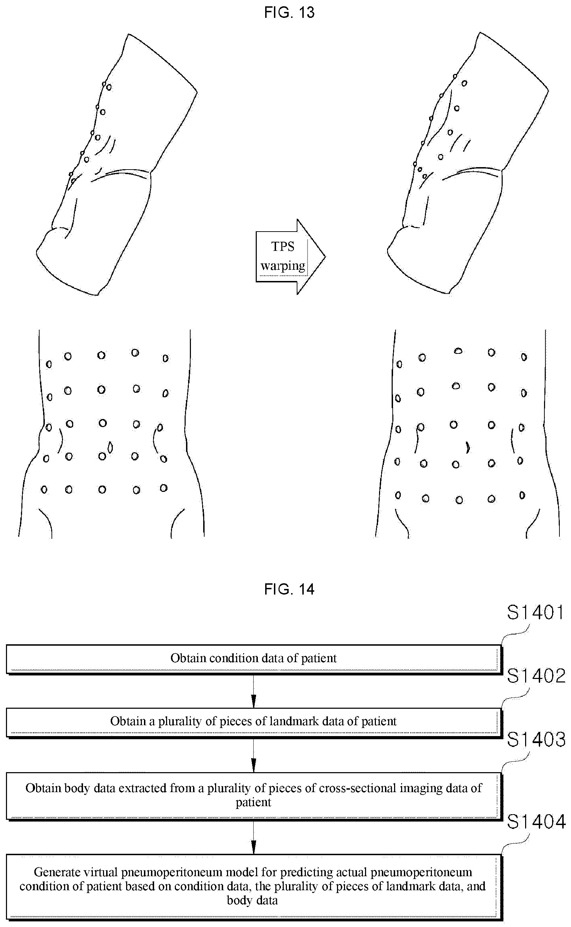

FIG. 13 is a drawing for describing generating a virtual pneumoperitoneum model according to the present disclosure.

Hereinafter, a description will be given the device 10 for generating the virtual pneumoperitoneum model of the patient according to the present disclosure with reference to FIGS. 1 to 13.

The device 10 according to the present disclosure may obtain condition data of the patient, may obtain a plurality of pieces of landmark data of the patient, may obtain body data extracted from a plurality of pieces of cross-sectional imaging data of the patient, and may generate a virtual pneumoperitoneum model for predicting an actual pneumoperitoneum condition of the patient based on the condition data, the plurality of pieces of landmark data, and the body data.

In detail, when performing minimally invasive surgery (e.g., laparoscopic surgery or robotic surgery), a medical team may perform surgery using a surgical instrument which enters a body through one or more trocar inserted into another position while checking a partial range in the body by means of a camera when enters the body through a trocar penetrating the body.

At this time, the medical team may inject predetermined gas (e.g., carbon dioxide) into the body when performing minimally invasive surgery (e.g., a space between abdominal walls when performing abdominal surgery) to secure a space in which the surgical instrument will move in the body.

The medical team wants to simulate surgery in advance before actual surgery to secure preparedness for various variables capable of being generated upon the actual surgery. As preparedness for this, virtual surgery simulation may be provided in the same virtual surgery environment as the actual surgery.

Because the minimally invasive surgery is performed while the medical team checks the partial area of the body using only the camera (i.e., an endoscope) inserted into the body, when the medical team practices with an image displayed in a completely different position or direction to proceed with actual surgery when proceeding with virtual simulation, no practice effect may be obtained at all because images provided to the medical team during actual surgery are completely different. Particularly, although performing modeling of a virtual body model to be the same as a physical condition of the patient upon body surgery, when the camera is entered in a different position or direction, the medical team may not obtain practice effects by practicing while watching completely different images.

As the shape of the body surface (e.g., abdomen) changes when a pneumoperitoneum condition is applied to the body of the patient, although the same position as the body surface in the pneumoperitoneum condition is specified on a 3D body mode which is not in the pneumoperitoneum condition, the angle at which the camera is inserted changes.

Thus, there is a need to implement a virtual pneumoperitoneum model to which the pneumoperitoneum condition is applied to be the same as during actual surgery when generating a virtual body model for performing surgery simulation.

Hereinafter, a description will be given of a method for generating the virtual pneumoperitoneum model, which may be performed by the device 10 for generating the virtual pneumoperitoneum model of the patient.

The device 10 may generate the virtual pneumoperitoneum model of the patient based on the condition data of the patient, the landmark data, and the body data, thus predicting an actual pneumoperitoneum condition of the patient at high accuracy.

In detail, the device 10 may generate the virtual pneumoperitoneum model of the patient based on the condition data, such as an age of the patient, a gender of the patient, a height of the patient, a weight of the patient, a body mass index of the patient, and whether the patient has given birth, the body data, such as a ratio between a height and a width for a body of the patient, a skin circumference, a distance between the front of the body and the rear of the body, a fat area, and a muscle area, and the landmark data displayed on abdominal 3D imaging data of the patient before pneumoperitoneum, thus predicting the actual pneumoperitoneum condition of the patient at high accuracy.

Such a device 10 may include all of various devices capable of performing arithmetic processing and providing a user with the result of performing the arithmetic processing.

Herein, the device 10 may be in the form of a computer. In detail, the computer may include all of various devices capable of performing arithmetic processing and providing the user with the result of performing the arithmetic processing.

For example, the computer may correspond to a smartphone, a tablet personal computer (PC), a cellular phone, a personal communication service (PCS) phone, a synchronous/asynchronous international mobile telecommunication-2000 (IMT-2000) mobile phone, a palm PC, a personal digital assistant (PDA), or the like as well as a desktop PC or a note book. Furthermore, when a head mounted display (HMD) device includes a computing function, it may be the computer.

Furthermore, the computer may correspond to a server which receives a request from a client and performs information processing.

The device 10 may include a communication unit 110, a memory 120, and a processor 130. Herein, the device 10 may include less or more components than the components shown in FIG. 1.

The communication unit 110 may include one or more modules capable of performing wireless communication between the device 10 and an external device (not shown), between the device 10 and an external server (not shown), or between the device 10 and a communication network (not shown).

Herein, the external device (not shown) may be medical imaging equipment which captures medical imaging data (hereinafter referred to as "abdominal 3D image data"). Herein, the medical imaging data may include all medical imaging capable of implementing the body of the patient as a 3D model.

Furthermore, the medical imaging data may include at least one of computed tomography (CT) imaging, magnetic resonance imaging (MRI), or positron emission tomography (PET) imaging.

Furthermore, the external server (not shown) may be a server which stores medical data for each patient for a plurality of patients.

Furthermore, the communication network (not shown) may transmit and receive various pieces of information between the device 10, the external device (not shown), and the external server (not shown). Herein, the communication network may include various types of communication networks and may use, for example, a wireless communication scheme, such as wireless local area network (WLAN), wireless-fidelity (Wi-Fi), wireless broadcast (Wibro), worldwide interoperability for microware access (WiMAX), or high speed downlink packet access (HSDPA), or a wired communication scheme, such as an Ethernet, xDSL (ADSL, VDSL), hybrid fiber coax (HFC), fiber to the curb (FTTC), or fiber to the home (FTTH).

Meanwhile, the communication network is not limited to the above-mentioned communication schemes, which may include all types of communication schemes which are well known or will be developed in the future other than the above-mentioned communication schemes.

The communication unit 110 may include one or more modules which connect the device 10 with one or more networks.

The memory 120 may store data for supporting various functions of the device 10. The memory 120 may store a plurality of application programs (or applications) run in the device 10, pieces of data for an operation of the device 10, or instructions. At least some of such application programs may be present for a basic function of the device 10. Meanwhile, the application program may be stored in the memory 120, may be installed on the device 10, and may be run to perform an operation (or function) of the device 10 by the processor 130.

Furthermore, the memory 120 may include a plurality of processes for generating the virtual pneumoperitoneum model according to the present disclosure. Herein, the plurality of processes will be described below when describing the operation of the processor 130.

The processor 130 may generally control the overall operation of the device 10 other than an operation associated with the application program. The processor 130 may process a signal, data, information, or the like input or output through the components described above or may run the application program stored in the memory 120, thus providing or processing information or a function suitable for the user.

Furthermore, the processor 130 may control at least some of the components described above with reference to FIG. 1 to run the application program stored in the memory 120. In addition, the processor 130 may combine and operate at least two or more of the components included in the device 10 to run the application program.

The processor 130 may obtain the condition data of the patient. Herein, the processor 130 may obtain the condition data of the patient from the external server (not shown) or the memory 120 based on a first process among the plurality of processes.

Herein, the condition data may include data about at least one of an age of the patient, a gender of the patient, a height of the patient, a weight of the patient, a body mass index of the patient, or whether the patient has given birth.

Such condition data may be clinical information capable of affecting formation of pneumoperitoneum, when performing pneumoperitoneum of the abdomen of the patient.

The processor 130 may obtain a plurality of pieces of landmark data of the patient. Herein, the processor 130 may obtain the plurality of pieces of landmark data of the patient based on a second process among the plurality of processes. Herein, the plurality of pieces of landmark data may be to be displayed on an abdominal 3D imaging data of the patient. The plurality of pieces of landmark data may be data in which landmarks are mapped to the abdominal 3D imaging data of the patient or may be coordinate data of each of the landmarks.

The plurality of pieces of landmark data may be used for the purpose of inflating an abdomen around the corresponding landmark to form pneumoperitoneum or may be used as reference points capable of measuring how much it has been deformed after the pneumoperitoneum is formed.

In detail, the processor 130 may generate one piece of reference landmark data at a predetermined position with respect to a navel of the patient based on the abdominal 3D imaging data of the patient and may additionally generate the plurality of pieces of landmark data on the basis of the reference landmark data.

Herein, the plurality of pieces of landmark data may be directly generated by the user by means of the device 10 or may be automatically generated at a predetermined interval (e.g., 5 cm) around the navel. Herein, the user may be a medical professional, which may be a doctor, a nurse, a clinical pathologist, and a medical imaging specialist, or the like and may be a technician who repairs a medical device, but not limited thereto.

Referring to FIG. 2, the processor 130 may generate one piece of reference landmark data 201 at a predefined position with respect to the navel of a first patient based on abdominal 3D imaging data of the first patient.

Thereafter, the processor 130 may additionally generate the plurality of pieces of landmark data on the basis of the reference landmark data 201.

Herein, the processor 130 may additionally generate the plurality of pieces of landmark data at predetermined equal intervals on the basis of the reference landmark data 201, but not necessarily limited thereto.

The processor 130 may obtain body data extracted from a plurality of pieces of cross-sectional imaging data of the patient. Herein, the processor 130 may obtain the body data extracted from the plurality of pieces of cross-sectional imaging data based on a third process among the plurality of processes.

Herein, the plurality of pieces of cross-sectional imaging data may be imaging data for a cross section of any one position among respective positions at which the plurality of pieces of landmark data are displayed or may be imaging data for a cross section of a portion which connects two or more locations into one.

Herein, the body data may include at least one of a ratio between a height and a width for the body of the patient in the plurality of pieces of cross-sectional imaging data, a skin circumference, a distance between the front of the body and the rear of the body, a fat area, or a muscle area.

Herein, the distance between the front of the body and the rear of the body may be a distance from the front of a navel part to the rear of a back part.

Referring to FIG. 3, the processor 130 may obtain a ratio between a height 301 and a width 302 for the body of the patient based on the plurality of pieces of cross-sectional imaging data generated in the above process. Herein, the ratio between the height 301 and the width 302 may vary for each position at which the cross-sectional imaging data is obtained. Alternatively, the ratio between the height 301 and the width 302 may vary for each patient.

Referring to FIG. 4, the processor 130 may obtain a skin circumference 401 for the body of the patient based on the plurality of pieces of cross-sectional imaging data. Herein, the skin circumference 401 for the body of the patient may vary for each position at which the cross-sectional imaging data is obtained. Alternatively, the skin circumference 401 for the body of the patient may vary for each patient.

Referring to FIG. 5, the processor 130 may obtain a distance between the front of the body and the rear of the body for the body of the patient based on the plurality of pieces of cross-sectional imaging data. Herein, the distance between the front of the body and the rear of the body for the body of the patient may vary for each patient.

Referring to FIG. 6, the processor 130 may obtain a fat area 601 for the body of the first patient based on the plurality of pieces of cross-sectional imaging data by means of a fat extraction model included in the memory 120. Herein, the fat area 601 for the body of the first patient may vary for each position at which the cross-sectional imaging data is obtained.

Furthermore, the processor 130 may obtain a fat area 602 for a body of a second patient based on the plurality of pieces of cross-sectional imaging data by means of the fat extraction model. Herein, the fat area 602 for the body of the second patient may vary for each position at which the cross-sectional imaging data is obtained.

The fat area for the body of the patient may vary for each patient.

Herein, the fat area may include at least one of a visceral fat area or a subcutaneous fat area.

Herein, the fat extraction model may be a machine learning model trained by specifying only a fat area as a region of interest on the abdominal 3D imaging data.

The machine learning model may include, but is not necessarily limited to, a convolutional neural network (CNN). The machine learning model may be formed of various structures of neural networks.

Referring to FIG. 7, the processor 130 may obtain a muscle area 701 for the body of the first patient based on the plurality of pieces of cross-sectional imaging data by means a muscle extraction model included in the memory 120. Herein, the muscle area 701 for the body of the first patient may vary for each position at which the cross-sectional imaging data is obtained.

Furthermore, the processor 130 may obtain a muscle area 702 for the body of the second patient based on the plurality of pieces of cross-sectional imaging data by means of the muscle extraction model. Herein, the muscle area 702 for the body of the second patient may vary for each position at which the cross-sectional imaging data is obtained.

The muscle area for the body of the patient may vary for each patient.

Herein, the muscle extraction model may be a machine learning model trained by specifying only a muscle area as a region of interest on the abdominal 3D imaging data.

The machine learning model may include, but is not necessarily limited to, a convolutional neural network (CNN). The machine learning model may be formed of various structures of neural networks.

The processor 130 may generate a virtual pneumoperitoneum model for predicting an actual pneumoperitoneum condition of the patient based on the condition data, the plurality of pieces of landmark data, and the body data.

Herein, the processor 130 may generate the virtual pneumoperitoneum model for predicting the actual pneumoperitoneum condition of the patient based on a fourth process among the plurality of processes.

In detail, the processor 130 may generate the virtual pneumoperitoneum model by means of the plurality of previously stored pneumoperitoneum shape classes or the machine learning model based on the condition data, the plurality of pieces of landmark data, and the body data.

First of all, the processor 130 may select specific pneumoperitoneum shape data with a highest matching rate with the condition data, the landmark data, and the body data among the plurality of pieces of previously stored pneumoperitoneum shape data based on a first algorithm to generate the virtual pneumoperitoneum model.

Herein, when the condition data, the body data, and the pneumoperitoneum shape data for every a plurality of existing patients increase, the processor 130 may cluster such pieces of data to generate a plurality of pneumoperitoneum shape classes.

Herein, the pneumoperitoneum shape data may be actual pneumoperitoneum shape data of the patient, which is generated by performing pneumoperitoneum for each actual existing patient and three-dimensionally scanning the body of the patient.

In other words, referring to FIG. 8, the processor 130 may cluster the condition data, the body data, and the pneumoperitoneum shape data for every a plurality of existing patients by means of a k-mean clustering algorithm to generate k pneumoperitoneum shape classes (e.g., when k = 3).

Herein, the algorithm used for clustering may include at least one of the k-mean clustering algorithm, an adaptive breaking detection clustering algorithm, or a Hough Transform algorithm.

In other words, when the condition data, the body data, and the pneumoperitoneum shape data for each existing patient increase, the processor 130 may generate the plurality of pneumoperitoneum shape classes by means of a specific algorithm (e.g., the k-mean clustering algorithm).

Herein, a plurality of pieces of pneumoperitoneum shape data shown in FIG. 9 may be pneumoperitoneum shape data for each existing patient included in the first class (or class 1), when the pneumoperitoneum shape classes are three. In other words, a plurality of existing patient included in the first class may be patients classified as one class in which condition data, landmark data, and body data are similar to each other.

Furthermore, a plurality of pieces of pneumoperitoneum shape data shown in FIG. 10 may be pneumoperitoneum shape data for each existing patient included in the second class (or class 2), when the pneumoperitoneum shape classes are three. In other words, a plurality of existing patient included in the second class may be patients classified as one class in which condition data, landmark data, and body data are similar to each other.

Furthermore, a plurality of pieces of pneumoperitoneum shape data shown in FIG. 11 may be pneumoperitoneum shape data for each existing patient included in the third class (or class 3), when the pneumoperitoneum shape classes are three. In other words, a plurality of existing patient included in the third class may be patients classified as one class in which condition data, landmark data, and body data are similar to each other.

Thus, when data increases as pieces of data for every a plurality of existing patients are clustered for each class according to a similarity, the processor 130 may first find the most similar class and may then select pneumoperitoneum shape data which is most similar to a new patient in the most similar class, thus quickly selecting the pneumoperitoneum shape data.

Thus, referring to FIG. 12, the processor 130 may select a specific pneumoperitoneum shape class with a highest similarity to medical imaging data to which the condition data, the body data, and the landmark data are mapped among the plurality of previously stored pneumoperitoneum shape classes.

In detail, the processor 130 may select the specific pneumoperitoneum shape class with the highest similarity to the condition data, the landmark data, and the body data for the patient based on a support vector machine (SVM) classification model among the plurality of pneumoperitoneum shape classes.

The processor 130 may select the specific pneumoperitoneum shape data in the selected specific pneumoperitoneum shape class, based on the first algorithm.

In detail, the processor 130 may select the specific pneumoperitoneum shape data in which the condition data, the body data, and the specific pneumoperitoneum shape data for the patient are most similar in the selected specific pneumoperitoneum shape class, based on a cosine similarity algorithm.

In other words, the processor 130 may select the specific pneumoperitoneum shape data in which a cosine similarity for the condition data, the body data, and the specific pneumoperitoneum shape data for the patient is highest in the selected specific pneumoperitoneum shape class, based on a cosine similarity algorithm.

Herein, an equation for the cosine similarity algorithm may be explained as Equation 1 below.

[Equation 1]

$$\text{Cosine similarity} = \frac{A \cdot B}{\|A\|\|B\|} = \frac{\sum_{i=1}^{n} A_i B_i}{\sqrt{\sum_{i=1}^{n} A_i^2} \sqrt{\sum_{i=1}^{n} B_i^2}}$$

Herein, A may denote the vector for the condition data and the condition data for the patient data, and B may denote the vector for the condition data and the body data for the data in the selected specific class.

Thus, a denominator part may indicate a magnitude of each vector, and a numerator part may be calculated by multiplication between the two vectors. When the two vectors are identical to each other, the cosine similarity may have a value of "1". When the two vectors are different from each other, the cosine similarity may have a value of "0".

Next, the processor 130 may generate the virtual pneumoperitoneum model based on the condition data, the landmark data, and the body data by means of the machine learning model.

The machine learning model may include, but is not necessarily limited to, a convolutional neural network (CNN). The machine learning model may be formed of various structures of neural networks.

In detail, the processor 130 may calculate position information after pneumoperitoneum in the landmark data for the patient by means of the machine learning model.

In more detail, the processor 130 may calculate position information after pneumoperitoneum in the landmark data for the patient, based on a regression model among the machine learning models.

Herein, an equation for the regression model may be explained as Equation 2 below.

[Equation 2]

$$\hat{y} = \beta_0 + \sum_{i=1}^{p} \beta_i x_i$$

Herein, y may denote the position information after pneumoperitoneum in the landmark data, x (independent variable) may denote the condition data and the body data, p may denote the total number of the condition data and the body data, and $\beta$ may denote the information indicating the Influence on x (the independent variable).

In detail, the processor 130 may calculate $\beta$ based on training data of the regression model.

In other words, because of knowing both of x and y values through the training data, the processor 130 may calculate $\beta$. Herein, y (dependent variable) may be a motion vector before and after pneumoperitoneum in pieces of training data.

Thereafter, y (position information) may be predicted by calculating x for new data and calculating β together.

The processor 130 may generate a body surface of the patient through thin plate spline (TPS) warping based on the position information after pneumoperitoneum, thus outputting the virtual pneumoperitoneum model.

Herein, referring to FIG. 13, the left upper end indicates the side of a virtual model generated based on the abdominal 3D imaging data for the patient. A plurality of pieces of landmark data may be displayed on the virtual model.

Herein, the left lower end indicates the front of the virtual model for the patient. The plurality of pieces of landmark data may be displayed on the virtual model.

Furthermore, the right upper end indicates the virtual pneumoperitoneum model after pneumoperitoneum for the patient by means of the machine learning model. Positions after pneumoperitoneum in the plurality of pieces of landmark data may be displayed on the virtual pneumoperitoneum model.

Herein, the right lower end indicates the front of the virtual pneumoperitoneum model after pneumoperitoneum for the patient. Positions after pneumoperitoneum in the plurality of pieces of landmark data may be displayed on the virtual pneumoperitoneum model after pneumoperitoneum.

When comparing the left of FIG. 13 with the right of FIG. 13, the virtual model indicating a condition of the patient before pneumoperitoneum and the virtual pneumoperitoneum model indicating a condition of the patient after pneumoperitoneum may have changes in positions of the plurality of pieces of landmark data. Because pneumoperitoneum is performed in various forms for each patient according to the change in position according to the pneumoperitoneum, the positions of the plurality of pieces of landmark data may change to various positions.

FIG. 14 is a flowchart illustrating a process of generating a virtual pneumoperitoneum model of a patient according to the present disclosure. Herein, an operation of a processor 130 may be performed by a server 10.

In operation S1401, the processor 130 may obtain condition data of the patient.

In detail, the processor 130 may obtain the condition data of the patient from an external server (not shown) or a memory 120.

Herein, the condition data may include data about at least one of an age of the patient, a gender of the patient, a height of the patient, a weight of the patient, a body mass index of the patient, or whether the patient has given birth.

In operation S1402, the processor 130 may obtain a plurality of pieces of landmark data of the patient.

Herein, the plurality of pieces of landmark data may be displayed on abdominal 3D imaging data of the patient. The plurality of pieces of landmark data may be data in which landmarks are mapped to the abdominal 3D imaging data of the patient or may be coordinate data of each of the landmarks.

In operation S1403, the processor 130 may obtain body data extracted from a plurality of pieces of cross-sectional imaging data of the patient.

Herein, the plurality of pieces of cross-sectional imaging data may be a cross section of positions at which the plurality of pieces of landmark data are displayed.

Furthermore, the body data may include at least one of a ratio between a height and a width for a body of the patient in the plurality of pieces of cross-sectional imaging data, a skin circumference, a distance between the front of the body and the rear of the body (or an A-P direction distance), a fat area, or a muscle area.

The processor 130 may respectively obtain the fat area and the muscle area based on the plurality of pieces of cross-sectional imaging data by means of a fat extraction model and a muscle extraction model.

In operation S1404, the processor 130 may generate a virtual pneumoperitoneum model for predicting an actual pneumoperitoneum condition of the patient based on the condition data, the plurality of pieces of landmark data, and the body data.

First of all, the processor 130 may select specific pneumoperitoneum shape data with a highest matching rate with the condition data, the landmark data, and the body data among a plurality of pieces of previously stored pneumoperitoneum shape data based on a first algorithm to generate the virtual pneumoperitoneum model.

Herein, the processor 130 may select the specific pneumoperitoneum shape class with a highest similarity to the condition data, the landmark data, and the body data for the patient for the patient based on a support vector machine (SVM) classification model among a plurality of pneumoperitoneum shape classes.

The processor 130 may select the specific pneumoperitoneum shape data in the selected specific pneumoperitoneum shape class, based on the first algorithm.

In operation S1404, the processor 130 may generate the virtual pneumoperitoneum model based on the condition data, the landmark data, and the body data by means of a machine learning model.

In detail, the processor 130 may calculate position information after pneumoperitoneum in the landmark data for the patient by means of the machine learning model.

The processor 130 may generate a body surface of the patient through thin plate spline (TPS) warping based on the position information after pneumoperitoneum, thus outputting the virtual pneumoperitoneum model.

FIG. 14 illustrates that operations S1401 to S1404 are sequentially executed, but this only illustratively describes the technical scope of the embodiment. Because a person having ordinary skill in the art to which the embodiment pertains changes and executes the order described in FIG. 14 in the range which does not depart from the essential characteristic of the embodiment or executes one or more of operations S1401 to S1404 in parallel to apply various corrections and modifications, FIG. 14 is not limited to the time series order.

The above-mentioned method according to an embodiment of the present disclosure may be combined with a computer which is hardware and may be stored in a medium to be implemented as a program (or application) to be executed. Here, the computer may be the device 10 described above.

For the computer to read the program and execute the methods implemented with the program, the above-mentioned program may include a code coded into a computer language such as C, C++, Java, or a machine language readable through a device interface of the computer by a processor (CPU) of the computer. Such a code may include a functional code associated with a function and the like defining functions necessary for executing the methods and may include a control code associated with an execution procedure necessary for the processor of the computer to execute the functions according to a procedure. Further, such a code may further include a code associated with memory reference about whether additional information or media necessary for the processor of the computer to execute the functions is referred at any location (address number) of an internal or external memory of the computer. Further, if it is necessary for the processor of the computer to communicate with any computer or server located in a remote place to execute the functions, the code may further include a communication related code about how communication is performed with any computer or server located in a remote place using a communication module of the computer and whether to transmit and receive any information or media upon communication.

Operations of the method or algorithm described in connection with an embodiment of the present disclosure may be directly implemented in hardware, may be implemented with a software module executed by hardware, or may be implemented by a combination of the hardware and the software module. The software module may reside in a random access memory (RAM), a read only memory (ROM), an erasable programmable ROM (EPROM), an electrically erasable programmable ROM (EEPROM), a flash memory, a hard disk, a removable disk, a CD-ROM, or a computer-readable recording medium well known in the art to which the present disclosure pertains.

Although an embodiment of the present disclosure is described with reference to the accompanying drawings, it will be understood by those skilled in the art to which the present disclosure pertains that the present disclosure can be carried out in other detailed forms without changing the scope or the essential features of the present disclosure. Therefore, the embodiments described above are provided by way of example in all aspects, and should be construed not to be restrictive.

## Claims

1. A method for generating a virtual pneumoperitoneum model of a patient, which is performed by a device (10), the method comprising:

    obtaining condition data of the patient, the condition data including data about at least one of an age of the patient, a gender of the patient, a height of the patient, a weight of the patient, a body mass index of the patient, or whether the patient has given birth;
    obtaining a plurality of pieces of landmark data of the patient, the plurality of pieces of landmark data being displayed on an abdominal 3D imaging data of the patient;
    obtaining body data extracted from a plurality of pieces of cross-sectional imaging data of the patient, **characterized in that**
    the plurality of pieces of cross-sectional imaging data are a cross section of positions at which the plurality of pieces of landmark data are displayed and the body data includes at least one of a ratio between a height (301) and a width (302) for a body of the patient in the plurality of pieces of cross-sectional imaging data, a skin circumference (401), a distance between the front of the body and the rear of the body, a fat area (601, 602), or a muscle area (701, 702); and
    generating the virtual pneumoperitoneum model for predicting an actual pneumoperitoneum condition of the patient based on the condition data, the plurality of pieces of landmark data, and the body data.

2. The method of claim 1, wherein the obtaining of the plurality of pieces of landmark data includes:

    generating one piece of reference landmark data (201) at a predetermined position with respect to a navel of the patient based on the abdominal 3D imaging data of the patient; and
    additionally generating the plurality of pieces of landmark data on the basis of the reference landmark data (201).

3. The method of claim 1, wherein the generating of the virtual pneumoperitoneum model includes:

selecting specific pneumoperitoneum shape data with a highest matching rate with the condition data, the landmark data, and the body data among a plurality of pieces of previously stored pneumoperitoneum shape data based on a first algorithm.

4. The method of claim 3, wherein the generating of the virtual pneumoperitoneum model includes:

selecting a specific pneumoperitoneum shape class with a highest similarity with the condition data, the landmark data, and the body data among a plurality of previously stored pneumoperitoneum shape classes;
selecting the specific pneumoperitoneum shape data in the selected specific pneumoperitoneum shape class, based on the first algorithm, and
wherein the plurality of previously stored pneumoperitoneum shape classes are generated by clustering the condition data, the body data, and the pneumoperitoneum shape data for every a plurality of existing patients.

5. The method of claim 1, wherein the generating of the virtual pneumoperitoneum model includes:
generating the virtual pneumoperitoneum model based on the condition data and the body data by means of a machine learning model.

6. The method of claim 5, wherein the generating of the virtual pneumoperitoneum model includes:

calculating position information after pneumoperitoneum in the landmark data for the patient by means of the machine learning model;
generating a body surface of the patient based on the position information after pneumoperitoneum and outputting the virtual pneumoperitoneum model,
wherein the machine learning model is trained based on a training dataset constructed by constructing a training dataset based on the condition data, the body data, the landmark data, and landmark data after pneumoperitoneum for every a plurality of existing patients, and

wherein the landmark data after pneumoperitoneum is obtained based on an actual pneumoperitoneum result when performing surgery on an existing patient.

7. The method of claim 1, wherein the obtaining of the body data includes:

obtaining the fat area (601, 602) based on the plurality of pieces of cross-sectional imaging data by means of a fat extraction model; and
obtaining the muscle area (701, 702) based on the plurality of pieces of cross-sectional imaging data by means of a muscle extraction model,
wherein the fat extraction model is a machine learning model trained by specifying only a fat area as a region of interest on abdominal medical imaging data, and
wherein the muscle extraction model is a machine learning model trained by specifying only a muscle area as a region of interest on the abdominal medical imaging data.

8. A computer program being combined with a computer which is hardware and being stored in a computer-readable storage medium to execute a method for generating a virtual pneumoperitoneum model of a patient in claim 1.

9. A device (10) for generating a virtual pneumoperitoneum model of a patient, the device (10) comprising:

a memory (120) storing a plurality of processes for generating the virtual pneumoperitoneum model of the patient; and
a processor (130) configured to generate the virtual pneumoperitoneum model of the patient based on the plurality of processes,
wherein the processor (130) is configured to:

obtain condition data of the patient, the condition data including data about at least one of an age of the patient, a gender of the patient, a height of the patient, a weight of the patient, a body mass index of the patient, or whether the patient has given birth;
obtain a plurality of pieces of landmark data of the patient, the plurality of pieces of landmark data being displayed on an abdominal 3D imaging data of the patient;
obtain body data extracted from a plurality of pieces of cross-sectional imaging data of the patient,

**characterized in that**

the plurality of pieces of cross-sectional imaging data are a cross section of positions at which the plurality of pieces of landmark data are displayed and the body data includes at least one of a ratio between a height (301) and a width (302) for a body of the patient in the plurality of pieces of cross-sectional imaging data, a skin circumference (401), a distance between the front of the body and the rear of the body, a fat area (601, 602), or a muscle area (701, 702); and

generate the virtual pneumoperitoneum model for predicting an actual pneumoperitoneum condition of the patient based on the condition data, the plurality of pieces of landmark data, and the body data.

**10.** The device (10) of claim 9, wherein the processor (130) is configured to:

when obtaining the landmark data,
generate one piece of reference landmark data (201) at a predetermined position with respect to a navel of the patient based on the abdominal 3D imaging data of the patient; and
additionally generate the plurality of pieces of landmark data on the basis of the reference landmark data (201).

**11.** The device (10) of claim 9, wherein the processor (130) is configured to:

when generating the virtual pneumoperitoneum model,
select specific pneumoperitoneum shape data with a highest matching rate with the condition data, the landmark data, and the body data among a plurality of pieces of previously stored pneumoperitoneum shape data based on a first algorithm.

**12.** The device (10) of claim 11, wherein the processor (130) is configured to:

when generating the virtual pneumoperitoneum model,
select a specific pneumoperitoneum shape class with a highest similarity with the condition data, the landmark data, and the body data among a plurality of previously stored pneumoperitoneum shape classes; and
select the specific pneumoperitoneum shape data in the selected specific pneumoperitoneum shape class, based on the first algorithm, and
wherein the plurality of previously stored pneumoperitoneum shape classes are generated by clustering the condition data, the body data, and the pneumoperitoneum shape data for every a plurality of existing patients.

**13.** The device (10) of claim 9, wherein the processor (130) is configured to:

when generating the virtual pneumoperitoneum model,
generate the virtual pneumoperitoneum model based on the condition data and the body data by means of a machine learning model.

**14.** The device (10) of claim 13, wherein the processor (130) is configured to:

when generating the virtual pneumoperitoneum model,
calculate position information after pneumoperitoneum in the landmark data for the patient by means of the machine learning model; and
generate a body surface of the patient based on the position information after pneumoperitoneum and output the virtual pneumoperitoneum model, and
wherein the machine learning model is trained based on a training dataset constructed by constructing a training dataset based on the condition data, the body data, the landmark data, and landmark data after pneumoperitoneum for every a plurality of existing patients, and
wherein the landmark data after pneumoperitoneum is obtained based on an actual pneumoperitoneum result when performing surgery on an existing patient.

**15.** The device (10) of claim 9, wherein the processor (130) is configured to:

when obtaining the body data,
obtain the fat area (601, 602) based on the plurality of pieces of cross-sectional imaging data by means of a fat extraction model; and
obtain the muscle area (701, 702) based on the plurality of pieces of cross-sectional imaging data by means of a muscle extraction model,
wherein the fat extraction model is a machine learning model trained by specifying only a fat area as a region of interest on abdominal medical imaging data, and
wherein the muscle extraction model is a machine learning model trained by specifying only a muscle area as a region of interest on the abdominal medical imaging data.

**Patentansprüche**

1. Verfahren zur Erzeugung eines virtuellen Pneumoperitoneum-Modells eines Patienten, das von einer Vorrichtung (10) durchgeführt wird, wobei das Verfahren die folgenden Schritte aufweist:

   Erfassen von Zustandsdaten des Patienten, wobei die Zustandsdaten Daten über das Alter des Patienten und/oder das Geschlecht des Patienten und/oder der Größe des Patienten und/oder dem Gewicht des Patienten und/oder dem Body-Mass-Index des Patienten und/oder darüber, ob der Patient entbunden hat, enthalten;
   Erfassen mehrerer Elemente von Markierungsdaten des Patienten, wobei die mehreren Elemente von Markierungsdaten auf einem abdominalen 3D-Bild des Patienten angezeigt werden;
   Erfassen von Körperdaten, die aus mehreren Elementen von Querschnittsbilddaten des Patienten extrahiert werden,
   **dadurch gekennzeichnet, dass**
   die mehreren Elemente von Querschnittsbilddaten einen Querschnitt von Positionen darstellen, an denen die mehreren Elemente von Markierungsdaten angezeigt werden,
   und die Körperdaten mindestens eines der folgenden Elemente umfassen: ein Verhältnis zwischen einer Höhe (301) und einer Breite (302) des Körpers des Patienten in den mehreren Elementen von Querschnittsbilddaten, einen Hautumfang (401), einen Abstand zwischen der Vorderseite des Körpers und der Rückseite des Körpers, einen Fettbereich (601, 602) oder einen Muskelbereich (701, 702); und
   Erzeugen des virtuellen Pneumoperitoneum-Modells zur Vorhersage eines tatsächlichen Pneumoperitoneum-Zustandes des Patienten auf der Grundlage der Zustandsdaten, der Markierungsdaten und der Körperdaten.

2. Verfahren nach Anspruch 1, wobei das Erfassen der mehreren Elemente von Markierungsdaten die folgenden Schritte aufweist:

   Erzeugen eines Elements von Referenzmarkierungsdaten (201) an einer vorbestimmten Position in Bezug auf einen Nabel des Patienten auf der Grundlage der abdominalen 3D-Bilddaten des Patienten; und
   zusätzliches Erzeugen der mehreren Markierungsdaten auf der Grundlage der Referenzmarkierungsdaten (201).

3. Verfahren nach Anspruch 1, wobei das Erzeugen des virtuellen Pneumoperitoneum-Modells den folgenden Schritt aufweist:
   Auswählen spezifischer Pneumoperitoneum-Formdaten mit einer höchsten Übereinstimmungsrate mit den Zustandsdaten, den Markierungsdaten und den Körperdaten aus mehreren Elementen zuvor gespeicherter Pneumoperitoneum-Formdaten auf der Grundlage eines ersten Algorithmus.

4. Verfahren nach Anspruch 3, wobei das Erzeugen des virtuellen Pneumoperitoneum-Modells die folgenden Schritte aufweist:

   Auswählen einer spezifischer Pneumoperitoneum-Formklasse mit einer größten Ähnlichkeit mit den Zustandsdaten, den Markierungsdaten und den Körperdaten aus mehreren Elementen zuvor gespeicherter Pneumoperitoneum-Formklassen;
   Auswählen der spezifischen Pneumoperitoneum-Formdaten in der ausgewählten spezifischen Pneumoperitoneum-Formklasse auf der Grundlage des ersten Algorithmus, und
   wobei die mehreren zuvor gespeicherten Pneumoperitoneum-Formklassen durch Clustern der Zustandsdaten, der Körperdaten und der Pneumoperitoneum-Formdaten für jeden von mehreren vorhandenen Patienten erzeugt werden.

5. Verfahren nach Anspruch 1, wobei das Erzeugen des virtuellen Pneumoperitoneum-Modells den folgenden Schritt aufweist:
   Erzeugen des virtuellen Pneumoperitoneum-Modells auf der Grundlage der Zustandsdaten und der Körperdaten mit Hilfe eines Maschinenlernmodells.

6. Verfahren nach Anspruch 5, wobei das Erzeugen des virtuellen Pneumoperitoneum-Modells die folgenden Schritte aufweist:

Berechnen von Positionsinformationen nach dem Pneumoperitoneum in den Markierungsdaten für den Patienten mit Hilfe des Maschinenlernmodells;

Erzeugen einer Körperoberfläche des Patienten auf der Grundlage der Positionsinformationen nach dem Pneumoperitoneum und Ausgeben des virtuellen Pneumoperitoneum-Modells,

wobei das Maschinenlernmodell auf der Grundlage eines Trainingsdatensatzes trainiert wird, der durch Konstruieren eines Trainingsdatensatzes auf der Grundlage der Zustandsdaten, der Körperdaten, der Markierungsdaten und der Markierungsdaten nach dem Pneumoperitoneum für jeden von mehreren vorhandenen Patienten konstruiert wird, und

wobei die Markierungsdaten nach dem Pneumoperitoneum auf der Grundlage eines tatsächlichen Pneumoperitoneum-Ergebnisses bei der Durchführung eines chirurgischen Eingriffs an einem bestehenden Patienten erhalten werden.

7. Verfahren nach Anspruch 1, wobei das Erfassen der Körperdaten die folgenden Schritte aufweist:

Erfassen der Fettfläche (601, 602) auf der Grundlage der mehreren Elemente von Querschnittsbilddaten mittels eines Fettextraktionsmodells; und

Erfassen der Muskelfläche (701, 702) auf der Grundlage der mehreren Elemente von Querschnittsbilddaten mittels eines Muskelextraktionsmodells;

wobei das Fettextraktionsmodell ein Maschinenlernmodell ist, das trainiert wird, indem nur ein Fettbereich als interessierende Region in medizinischen Bilddaten des Abdomens angegeben wird, und

wobei das Muskelextraktionsmodell ein Maschinenlernmodell ist, das trainiert wird, indem nur ein Muskelbereich als interessierende Region in den medizinischen Bilddaten des Abdomens angegeben wird.

8. Computerprogramm, das mit einem Computer kombiniert ist, bei dem es sich um Hardware handelt, und das in einem computerlesbaren Speichermedium gespeichert ist, um ein Verfahren zur Erzeugung eines virtuellen Pneumoperitoneum-Modells eines Patienten nach Anspruch 1 auszuführen.

9. Vorrichtung (10) zum Erzeugen eines virtuellen Pneumoperitoneum-Modells eines Patienten, wobei die Vorrichtung (10) umfasst:

einen Speicher (120), der mehrere Prozesse zur Erzeugung des virtuellen Pneumoperitoneum-Modells des Patienten speichert; und

einen Prozessor (130), der dazu ausgebildet ist, das virtuelle Pneumoperitoneum-Modell des Patienten auf der Grundlage der mehreren Prozesse zu erzeugen,

wobei der Prozessor (130) dazu ausgebildet ist,

Zustandsdaten des Patienten zu erfassen, wobei die Zustandsdaten Daten über das Alter des Patienten und/oder das Geschlecht des Patienten und/oder der Größe des Patienten und/oder dem Gewicht des Patienten und/oder dem Body-Mass-Index des Patienten und/oder darüber, ob der Patient entbunden hat, enthalten;

mehrere Elemente von Markierungsdaten des Patienten zu erfassen, wobei die mehreren Elemente von Markierungsdaten auf einem abdominalen 3D-Bild des Patienten angezeigt werden;

Körperdaten zu erfassen, die aus mehreren Elementen von Querschnittsbilddaten des Patienten extrahiert werden,

**dadurch gekennzeichnet, dass**

die mehreren Elemente von Querschnittsbilddaten einen Querschnitt von Positionen darstellen, an denen die mehreren Elemente von Markierungsdaten angezeigt werden,

und die Körperdaten mindestens eines der folgenden Elemente umfassen: ein Verhältnis zwischen einer Höhe (301) und einer Breite (302) des Körpers des Patienten in den mehreren Elementen von Querschnittsbilddaten, einen Hautumfang (401), einen Abstand zwischen der Vorderseite des Körpers und der Rückseite des Körpers, einen Fettbereich (601, 602) oder einen Muskelbereich (701, 702); und

das virtuelle Pneumoperitoneum-Modells zur Vorhersage eines tatsächlichen Pneumoperitoneum-Zustandes des Patienten auf der Grundlage der Zustandsdaten, der Markierungsdaten und der Körperdaten zu erzeugen.

10. Vorrichtung (10) nach Anspruch 9, wobei der Prozessor (130) dazu ausgebildet ist,

bei der Erfassung der Markierungsdaten,
ein Element von Referenzmarkierungsdaten (201) an einer vorbestimmten Position in Bezug auf einen Nabel des Patienten auf der Grundlage der abdominalen 3D-Bilddaten des Patienten zu erzeugen; und
zusätzlich die mehreren Markierungsdaten auf der Grundlage der Referenzmarkierungsdaten (201) zu erzeugen.

11. Vorrichtung (10) nach Anspruch 9, wobei der Prozessor (130) dazu ausgebildet ist,

bei der Erzeugung des virtuellen Pneumoperitoneum-Modells,
spezifische Pneumoperitoneum-Formdaten mit einer höchsten Übereinstimmungsrate mit den Zustandsdaten, den Markierungsdaten und den Körperdaten aus mehreren Elementen zuvor gespeicherter Pneumoperitoneum-Formdaten auf der Grundlage eines ersten Algorithmus auszuwählen.

12. Vorrichtung (10) nach Anspruch 11, wobei der Prozessor (130) dazu ausgebildet ist,

bei der Erzeugung des virtuellen Pneumoperitoneum-Modells,
eine spezifische Pneumoperitoneum-Formklasse mit einer größten Ähnlichkeit mit den Zustandsdaten, den Markierungsdaten und den Körperdaten aus mehreren Elementen zuvor gespeicherter Pneumoperitoneum-Formklassen auszuwählen;
die spezifischen Pneumoperitoneum-Formdaten in der ausgewählten spezifischen Pneumoperitoneum-Formklasse auf der Grundlage des ersten Algorithmus auszuwählen, und
wobei die mehreren zuvor gespeicherten Pneumoperitoneum-Formklassen durch Clustern der Zustandsdaten, der Körperdaten und der Pneumoperitoneum-Formdaten für jeden von mehreren vorhandenen Patienten erzeugt werden.

13. Vorrichtung (10) nach Anspruch 9, wobei der Prozessor (130) dazu ausgebildet ist,

bei der Erzeugung des virtuellen Pneumoperitoneum-Modells,
das virtuelle Pneumoperitoneum-Modell auf der Grundlage der Zustandsdaten und der Körperdaten mit Hilfe eines Maschinenlernmodells zu erzeugen.

14. Vorrichtung (10) nach Anspruch 13, wobei der Prozessor (130) dazu ausgebildet ist,

bei der Erzeugung des virtuellen Pneumoperitoneum-Modells,
Positionsinformationen nach dem Pneumoperitoneum in den Markierungsdaten für den Patienten mit Hilfe des Maschinenlernmodells zu berechnen; und
eine Körperoberfläche des Patienten auf der Grundlage der Positionsinformationen nach dem Pneumoperitoneum und Ausgeben des virtuellen Pneumoperitoneum-Modells zu erzeugen, und
wobei das Maschinenlernmodell auf der Grundlage eines Trainingsdatensatzes trainiert wird, der durch Konstruieren eines Trainingsdatensatzes auf der Grundlage der Zustandsdaten, der Körperdaten, der Markierungsdaten und der Markierungsdaten nach dem Pneumoperitoneum für jeden von mehreren vorhandenen Patienten konstruiert wird, und
wobei die Markierungsdaten nach dem Pneumoperitoneum auf der Grundlage eines tatsächlichen Pneumoperitoneum-Ergebnisses bei der Durchführung eines chirurgischen Eingriffs an einem bestehenden Patienten erhalten werden.

15. Vorrichtung (10) nach Anspruch 9, wobei der Prozessor (130) dazu ausgebildet ist, beim Erfassen der Körperdaten,

die Fettfläche (601, 602) auf der Grundlage der mehreren Elemente von Querschnittsbilddaten mittels eines Fettextraktionsmodells zu erfassen; und
die Muskelfläche (701, 702) auf der Grundlage der mehreren Elemente von Querschnittsbilddaten mittels eines Muskelextraktionsmodells zu erfassen;
wobei das Fettextraktionsmodell ein Maschinenlernmodell ist, das trainiert wird, indem nur ein Fettbereich als interessierende Region in medizinischen Bilddaten des Abdomens angegeben wird, und
wobei das Muskelextraktionsmodell ein Maschinenlernmodell ist, das trainiert wird, indem nur ein Muskelbereich als interessierende Region in den medizinischen Bilddaten des Abdomens angegeben wird.

## EP 4 379 740 B1

**Revendications**

1. Procédé de génération d'un modèle de pneumopéritoine virtuel d'un patient, qui est mis en œuvre par un dispositif (10), le procédé comprenant les étapes consistant à :

   obtenir des données de condition du patient, les données de condition comprenant des données se rapportant à au moins une composante parmi un âge du patient, un genre du patient, une taille du patient, un poids du patient, un indice de masse corporelle du patient, ou au fait que la patiente ait éventuellement accouché ;
   obtenir une pluralité d'éléments de données de point de repère du patient, les éléments de la pluralité d'éléments de données de point de repère étant affichés sur des données d'imagerie abdominale 3D du patient ;
   obtenir des données corporelles extraites à partir d'une pluralité d'éléments de données d'imagerie en coupe du patient,
   **caractérisé en ce que**
   les éléments de la pluralité d'éléments de données d'imagerie en coupe correspondent à une coupe transversale de positions au niveau desquelles sont affichés les éléments de la pluralité d'éléments de données de point de repère
   et
   les données corporelles comprennent au moins l'un d'un rapport entre une hauteur (301) et une largeur (302) d'un corps du patient dans les éléments de la pluralité d'éléments de données d'imagerie en coupe, d'une circonférence de peau (401), d'une distance entre le devant du corps et le derrière du corps, d'une zone de graisse (601, 602), ou d'une zone de muscle (701, 702) ; et
   générer le modèle de pneumopéritoine virtuel pour prédire une condition de pneumopéritoine réel du patient sur la base les données de condition, des éléments de la pluralité d'éléments de données de point de repère et des données corporelles.

2. Procédé selon la revendication 1, dans lequel l'étape d'obtention des éléments de la pluralité d'éléments de données de point de repère consiste à :

   générer un élément de données de point de repère de référence (201) à une position prédéterminée par rapport à un nombril du patient sur la base des données d'imagerie abdominale 3D du patient, et
   générer de plus les éléments de la pluralité d'éléments de données de point de repère sur la base des données de point de repère de référence (201).

3. Procédé selon la revendication 1, dans lequel l'étape de génération du modèle de pneumopéritoine virtuel consiste à : sélectionner des données de forme de pneumopéritoine spécifique affichant le taux de correspondance le plus élevé avec les données de condition, les données de point de repère et avec les données corporelles parmi les éléments d'une pluralité d'éléments de données de forme de pneumopéritoine mémorisées au préalable sur la base d'un premier algorithme.

4. Procédé selon la revendication 3, dans lequel l'étape de génération du modèle de pneumopéritoine virtuel consiste à :

   sélectionner une classe de forme de pneumopéritoine spécifique affichant une similarité la plus élevée avec les données de condition, les données de point de repère et avec les données corporelles parmi des classes d'une pluralité de classes de forme de pneumopéritoine mémorisées au préalable ;
   sélectionner les données de forme de pneumopéritoine spécifique dans la classe de forme de pneumopéritoine spécifique sélectionnée sur la base du premier algorithme, et
   dans lequel les classes de la pluralité de classes de forme de pneumopéritoine mémorisées au préalable sont générées par un regroupement des données de condition, des données corporelles et des données de forme de pneumopéritoine de chaque patient d'une pluralité de patients existants.

5. Procédé selon la revendication 1, dans lequel l'étape de génération du modèle de pneumopéritoine virtuel consiste à : générer le modèle de pneumopéritoine virtuel sur la base des données de condition et des données corporelles au moyen d'un modèle d'apprentissage automatique.

6. Procédé selon la revendication 5, dans lequel l'étape de génération du modèle de pneumopéritoine virtuel consiste à :

   calculer des informations de position après un pneumopéritoine dans les données de point de repère du patient au moyen du modèle d'apprentissage automatique ;

générer une surface corporelle du patient sur la base des informations de position après un pneumopéritoine et délivrer le modèle de pneumopéritoine virtuel,

dans lequel le modèle d'apprentissage automatique est entraîné sur la base d'un ensemble de données d'entraînement construit par une construction d'un ensemble de données d'entraînement sur la base des données de condition, des données corporelles, des données de point de repère et des données de point de repère après un pneumopéritoine de chaque patient d'une pluralité de patients existants, et

dans lequel les données de point de repère après un pneumopéritoine sont obtenues sur la base d'un résultat de pneumopéritoine réel lors de la pratique d'une intervention chirurgicale sur un patient existant.

7. Procédé selon la revendication 1, dans lequel l'étape d'obtention des données corporelles consiste à :

obtenir la zone de graisse (601, 602) sur la base des éléments de la pluralité d'éléments de données d'imagerie en coupe au moyen d'un modèle d'extraction de graisse ; et

obtenir la zone de muscle (701, 702) sur la base des éléments de la pluralité d'éléments de données d'imagerie en coupe au moyen d'un modèle d'extraction de muscle,

dans lequel le modèle d'extraction de graisse est un modèle d'apprentissage automatique entraîné par une spécification uniquement d'une zone de graisse en tant que région d'intérêt sur des données d'imagerie médicale abdominale, et

dans lequel le modèle d'extraction de muscle est un modèle d'apprentissage automatique entraîné par une spécification uniquement d'une zone de muscle en tant que région d'intérêt sur les données d'imagerie médicale abdominale.

8. Programme informatique combiné à un ordinateur qui constitue un matériel et qui est mémorisé dans un support d'informations lisible par ordinateur pour exécuter un procédé de génération d'un modèle de pneumopéritoine virtuel d'un patient selon la revendication 1.

9. Dispositif (10) de génération d'un modèle de pneumopéritoine virtuel d'un patient, le dispositif (10) comprenant :

une mémoire (120) maintenant une pluralité de processus de génération du modèle de pneumopéritoine virtuel du patient ; et

un processeur (130) configuré pour générer le modèle de pneumopéritoine virtuel du patient sur la base de la pluralité de processus,

dans lequel le processeur (130) est configuré pour :

obtenir des données de condition du patient, les données de condition comprenant des données se rapportant à au moins une composante parmi un âge du patient, un genre du patient, une taille du patient, un poids du patient, un indice de masse corporelle du patient, ou au fait que la patiente ait éventuellement accouché ;

obtenir une pluralité d'éléments de données de point de repère du patient, les éléments de la pluralité d'éléments de données de point de repère étant affichés sur des données d'imagerie abdominale 3D du patient ;

obtenir des données corporelles extraites à partir d'une pluralité d'éléments de données d'imagerie en coupe du patient,

**caractérisé en ce que**

les éléments de la pluralité d'éléments de données d'imagerie en coupe correspondent à une coupe transversale de positions au niveau desquelles sont affichés les éléments de la pluralité d'éléments de données de point de repère

et

les données corporelles comprennent au moins l'un d'un rapport entre une hauteur (301) et une largeur (302) d'un corps du patient dans les éléments de la pluralité d'éléments de données d'imagerie en coupe, d'une circonférence de peau (401), d'une distance entre le devant du corps et le derrière du corps, d'une zone de graisse (601, 602), ou d'une zone de muscle (701, 702) ; et

générer le modèle de pneumopéritoine virtuel pour prédire une condition de pneumopéritoine réel du patient sur la base les données de condition, des éléments de la pluralité d'éléments de données de point de repère et des données corporelles.

10. Dispositif (10) selon la revendication 9, dans lequel le processeur (130) est configuré pour :

lors de l'obtention des données de point de repère,

générer un élément de données de point de repère de référence (201) à une position prédéterminée par rapport à un nombril du patient sur la base des données d'imagerie abdominale 3D du patient ; et

générer de plus les éléments de la pluralité d'éléments de données de point de repère sur la base des données de point de repère de référence (201).

11. Dispositif (10) selon la revendication 9, dans lequel le processeur (130) est configuré pour :

lors de la génération du modèle de pneumopéritoine virtuel,

sélectionner des données de forme de pneumopéritoine spécifique affichant un taux de correspondance le plus élevé avec les données de condition, les données de point repère et avec les données corporelles parmi des éléments d'une pluralité d'éléments de données de forme de pneumopéritoine mémorisées au préalable sur la base d'un premier algorithme.

12. Dispositif (10) selon la revendication 11, dans lequel le processeur (130) est configuré pour :

lors de la génération du modèle de pneumopéritoine virtuel,

sélectionner une classe de forme de pneumopéritoine spécifique affichant une similarité la plus élevée avec les données de condition, les données de point de repère et avec les données corporelles parmi des classes d'une pluralité de classes de forme de pneumopéritoine mémorisées au préalable ; et

sélectionner les données de forme de pneumopéritoine spécifique dans la classe de forme de pneumopéritoine spécifique sélectionnée sur la base du premier algorithme, et

dans lequel les classes de la pluralité de classes de forme de pneumopéritoine mémorisées au préalable sont générées par un regroupement des données de condition, des données corporelles et des données de forme de pneumopéritoine de chaque patient d'une pluralité de patients existants.

13. Dispositif (10) selon la revendication 9, dans lequel le processeur (130) est configuré pour :

lors de la génération du modèle de pneumopéritoine virtuel,

générer le modèle de pneumopéritoine virtuel sur la base des données de condition et des données corporelles au moyen d'un modèle d'apprentissage automatique.

14. Dispositif (10) selon la revendication 13, dans lequel le processeur (130) est configuré pour :

lors de la génération du modèle de pneumopéritoine virtuel,

calculer des informations de position après un pneumopéritoine dans les données de point de repère du patient au moyen du modèle d'apprentissage automatique ; et

générer une surface corporelle du patient sur la base des informations de position après un pneumopéritoine et délivrer le modèle de pneumopéritoine virtuel, et

dans lequel le modèle d'apprentissage automatique est entraîné sur la base d'un ensemble de données d'entraînement construit par une construction d'un ensemble de données d'entraînement sur la base des données de condition, des données corporelles, des données de point de repère, et des données de point de repère après un pneumopéritoine de chaque patient d'une pluralité de patients existants, et

dans lequel les données de point de repère après un pneumopéritoine sont obtenues sur la base d'un résultat de pneumopéritoine réel lors de la pratique d'une intervention chirurgicale sur un patient existant.

15. Dispositif (10) selon la revendication 9, dans lequel le processeur (130) est configuré pour :

lors de l'obtention des données corporelles,

obtenir la zone de graisse (601, 602) sur la base des éléments de la pluralité d'éléments de données d'imagerie en coupe au moyen d'un modèle extraction de graisse ; et

obtenir la zone de muscle (701, 702) sur la base des éléments de la pluralité d'éléments de données d'imagerie en coupe au moyen d'un modèle d'extraction de muscle,

dans lequel le modèle d'extraction de graisse est un modèle d'apprentissage automatique entraîné par une spécification uniquement d'une zone de graisse en tant que région d'intérêt sur des données d'imagerie médicale abdominale, et

dans lequel le modèle d'extraction de muscle est un modèle d'apprentissage automatique entraîné par une spécification uniquement d'une zone de muscle en tant que région d'intérêt sur les données d'imagerie médicale

abdominale.

FIG. 1

FIG. 2

201

FIG. 3

301

302

FIG. 4

FIG. 5

FIG. 6

601

602

FIG. 7

701

702

## FIG. 8

## FIG. 9

Class 1

## FIG. 10

Class 2

FIG. 11

Class 3

FIG. 12

Select specific pneumoperitoneum shape data

Condition data, landmark data, and body data for first patient

Select specific pneumoperitoneum shape class based on SVM classification model

Select specific pneumoperitoneum shape data based on cosine similarity algorithm

FIG. 13

TPS
warping

FIG. 14

S1401

Obtain condition data of patient

S1402

Obtain a plurality of pieces of landmark data of patient

S1403

Obtain body data extracted from a plurality of pieces of cross-sectional imaging data of patient

S1404

Generate virtual pneumoperitoneum model for predicting actual pneumoperitoneum condition of patient based on condition data, the plurality of pieces of landmark data, and body data

**EP 4 379 740 B1**

REFERENCES CITED IN THE DESCRIPTION

**Patent documents cited in the description**

- KR 20200056855 A

- KR 102013814 B1